# EUROPEAN PATENT APPLICATION

(11) **EP 2 522 370 A1**
(43) Date of publication of application: **14.11.2012**
(21) Application number: 12167712.4
(22) Date of filing: 11.05.2012
(51) Int. Cl.: A61L 9/03, A01M 1/20

(54) **Vaporiser device**

(30) Priority: 13.05.2011 ES 201100557
(71) Applicant: Zyxtudio diseño e innovación SL, 46003 Valencia (ES)
(72) Inventor: Blasco Feo, Vicente D., 46003 VALENCIA (ES)
(74) Representative: Soler Lerma, Santiago

(57) **Abstract**

Improved vaporiser device with a heating cavity adequate for inserting and attaching a bottle and vaporising a substance contained in it and a heating cavity adequate for inserting and housing a pill impregnated with the material to vaporise and cause its vaporisation, the device being composed of two differentiated bodies wherein the first body has means for anchoring and attachment to a surface or to another body external to the device, such as electrical connection pins, and a second body joined to the first body by a union allowing it to swivel, so that depending on the relative position of one body with respect to the other the use of one heating cavity or the other is enabled.

## Description

As its title indicates, the present invention relates to a substance vaporiser device that is adequate for substances contained in bottles and those impregnated in a pill-like support.

The device comprises a first body provided with means for anchoring it, which can be the pins of the electrical plug, a support, and means allowing its swivelling union to a second body that comprises the heating means and means for housing and attaching the supports that supply the substance, bottle or pill, which swivels about the former such that depending on their relative position, it is possible to insert in the second body the bottle or pill, while not allowing them to coincide.

The field of the art to which it belongs is that of heating devices for evaporating aromatic substances or biocides.

### BACKGROUND OF THE INVENTION

Systems are known for evaporating substances to the atmosphere in which a wick is partially inserted in a bottle that contains the substance to evaporated, such that this substance, by capillarity, reaches the part of the wick that protrudes from the bottle, which is where the heat is applied in sufficient amount to evaporate the substance.

Also known are vaporisers that comprise a surface with means for heating it on which, either directly or indirectly, a pill impregnated in the substance to evaporate is placed, resulting in the gradual evaporation of this substance by the action of the heat.

There exist devices that allow housing a bottle with a wick or an impregnated pill as described above.

Utility Model ES1023655U relates to an electrical apparatus that can incorporate both a flask containing a liquid to vaporise with an impregnated wick that penetrates the heating chamber and means for placing a pill on this chamber.

ES2178444 also relates to a device for supplying vaporisable materials to a heater that allows said materials to be distributed in the air, considering different options for supplying the material to evaporate, both in tablet and in liquid form.

There are many patents seeking to protect specifically the appropriate heaters for generating and transferring the heat to a wick, a chimney shape, or a flat surface in the form of a tray.

A list of these includes but is not limited to ES2241799, ES2195597, ES2137114, ES2177164 and ES2112363, which do not affect the invention disclosed herein but reveal the interest in this type of device, as well as the technical solution hitherto supplied to achieve this result.

However, none of the cited patents provides a complete solution to the problem of preventing, while the device is being used to vaporise the substance supplied in one way, for example from the bottle, the vaporisation of the substance contained in the other, such as a pill, which in addition to implying an unnecessary consumption of electricity also implies an unnecessary use of the substances to vaporise.

In the devices described in patents ES2241799, ES2195597, ES2137114, ES2177164 and ES2112363 the main element is an essentially flat heater having an orifice that can be crossed by a wick that can protrude from the surface, such that when the device is occupied by a bottle it is complicated to install a pill, and vice versa. However, applying some pressure in both casings makes it possible for the bottle and pill to both be installed in the device, although they can be somewhat damaged when they are forcibly inserted.

### DESCRIPTION OF THE INVENTION

To solve the problems described above, a device is disclosed that can vaporise both substances contained in a bottle and substances that impregnate a pill in a non-concurrent manner, wherein the geometry and operation of the device prevents the two vaporisation forms from coexisting.

The device comprises two differentiated bodies joined to each other in a manner allowing one to swivel about the other, wherein the first body includes means for attaching it to a surface, such as the electrical plug pins themselves, a support, and rear extensions whose ends have external cylindrical protuberances disposed perpendicular in order to define the shaft about which the second body swivels, the second body comprising a casing that contains the heating means, the means for housing and, if applicable, attaching both the bottle and the pill containing the substance to vaporise, and the bushings that act jointly in the swivelling.

The device is preferably anchored to the wall, preferably by the electrical connection pins included in the first body.

Said first body presents rear extensions that end at the aforementioned cylindrical protuberances and which conform the shaft about which the second body swivels .

The second body comprises a casing, bushings appropriate for receiving the cylindrical protuberances that conform the shaft, a heating cavity allowing attaching bottles, preferably by their neck, and a heating cavity that can house pills, as well as the electronic devices used to control the heaters.

When it is in use this device will have a fixed part, the first body, and a swivelling part, the second body.

When the second, swivelling body is aligned with the rear extensions of the first body, the heating cavity in which a bottle can be fitted is exposed.

With the second body in this position, that is, aligned with the rear extensions of the first body, the same rear extensions of the first body partially occupy the heating cavity in which pills can be fitted, making it impossible for a pill and a bottle to coexist.

When the second body is perpendicular to the rear extensions of the first body, the mouth of the heating body in which bottles can be fitted is placed opposite and very near to the first body, preventing it from opening and thereby preventing a bottle from being fitted in it.

In this position, with the second body perpendicular to the extensions of the first body, the cavity in which a pill can be fitted is opened.

The heating elements are controlled by electronic means.

### BRIEF DESCRIPTION OF THE DRAWINGS

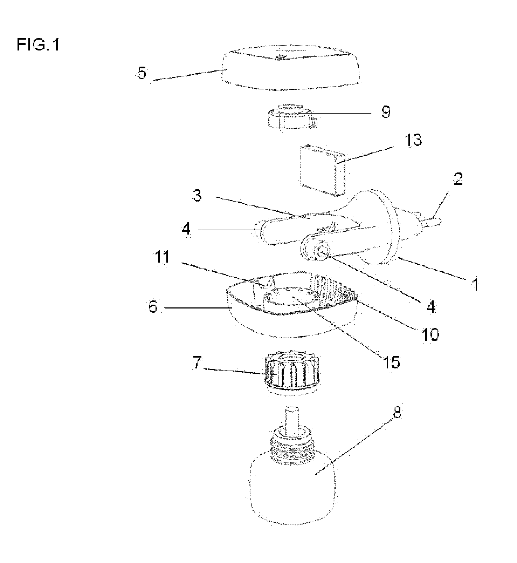

FIGURE 1 represents an exploded view of the device in the position in which the second body is aligned with the first body, thereby showing the first body (1) that comprises the electrical connection pins (2) and the rear extensions (3) that end in perpendicular cylindrical protuberances that conform a shaft (4), and the second body shown in an exploded view showing the top part of the casing (5) and its lower part (6), in which are seen both the orifice (15) that conforms the mouth of the heating cavity in which a bottle (8) an be fitted, which is composed of an attachment element (7) and a heating element (9), as well as the grooves (10) that enable the motion of the rear extensions (3) of the first body, the seat or bushing (11) of the shaft-like protuberances of said extensions and the means for heating the pill (13).

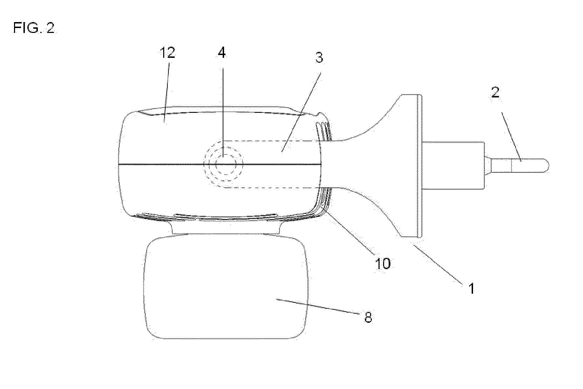

FIGURE 2 represents a side view of the device in the position in which the second body is aligned with the first body, showing the first body (1) with the electrical connection pins (2), and the rear extensions (3) that are introduced in the second body (12) that swivels about the protuberances that conform a shaft (4) located in the ends of the aforementioned extensions (3), wherein said extensions enter the second body through some grooves (10), also showing the bottle (8).

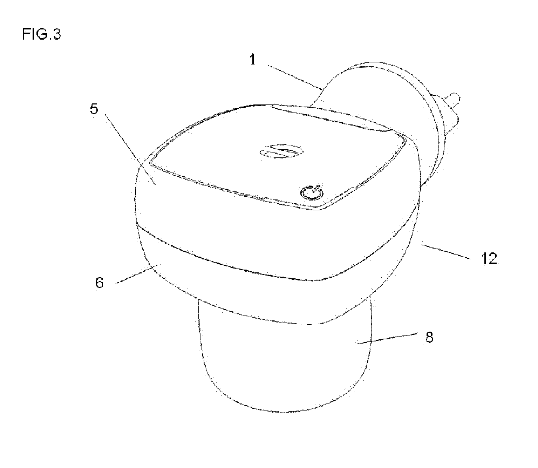

FIGURE 3 represents a perspective view of the device in the position in which the second body is aligned with the first body, revealing the casing (12) with its upper and lower parts (5) and (6), the inserted bottle (8) and the first body (1).

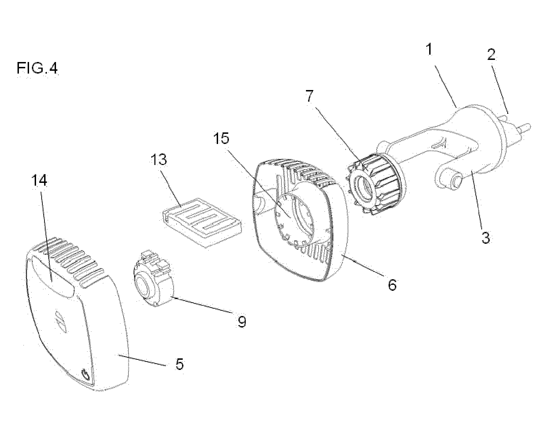

FIGURE 4 represents an exploded view of the device in the position in which the second body is perpendicular to the first body, showing the first body (1) with the electrical connection pins (2) and the rear extensions (3) described above, the upper part (5) and lower part (6) of the casing, the orifice (15) that provides access to the bottle heating cavity, the means for heating the bottle (9), the means for heating the pill (13), the groove through which the pill is inserted (14) and the means for attaching the bottle (7).

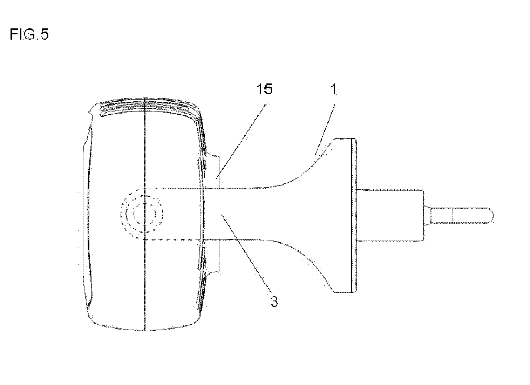

FIGURE 5 represents a side view of the device in the position in which the second body is perpendicular to the first body, showing that the orifice through which is inserted the bottle (15) is placed opposite and near the first body (1) so that it cannot be opened for normal use.

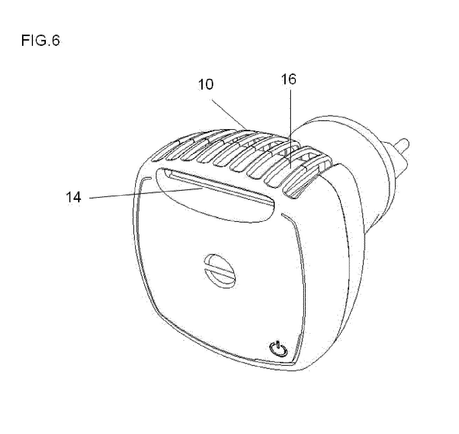

FIGURE 6 represents a perspective view of the device in which the second body is perpendicular to the first body, showing the grooves (10) of the second body, the groove through which the pill is inserted (14) and the pill (16) inside the device.

### DESCRIPTION OF AN EMBODIMENT OF THE INVENTION

Described below is a non-limiting example of an embodiment of the invention, so that other manufacturing alternatives will be included in it provided its characteristic elements are reproduced.

The device comprises two main bodies.

The first body (1) comprises the pins for connection to the electrical grid (2), which in turn act as anchoring means, and a support to which they are attached, extending from the rear of which are two extensions (3) that end at perpendicular cylindrical protuberances (4) that conform a shaft.

The second body, which swivels about the first body by the action of the shaft-like protuberances (4) and the bushings (11), comprises a casing (12) composed of an upper part (5) and a lower part (6) that has a groove (14) providing access to heating cavity for pills with means for heating the pill (13) and an orifice (15) providing access to a heating cavity for bottles composed of an attachment element (7) and a heating element (9).

The second body is aligned with the rear extensions of the first body, such that these extensions block the entry of the groove through which the pill (14) is introduced and partially occupy the space provided for the pill (16) making it impossible to insert a pill.

If there is a pill inside the second body from an earlier use, when the second body swivels to align itself with the rear extensions of the first body, the pill will be expelled as it will be pushed by the extensions when these move.

In this position, with the second body aligned with the rear extensions of the first body, the inlet orifice (15) to the bottle heating cavity will be in the bottom part and open to the outside, allowing to insert a suitable bottle (8), of the type that incorporate a protruding wick impregnated with the substance to vaporise, the bottle being attached to the device by the action of the attachment element (7).

In this position, when the heater (9) is turned on it will transmit heat to the wick of the bottle, evaporating the substance with which it is impregnated.

To use the device to vaporise substances impregnated in a pill, the device must be turned until the second body is perpendicular to the rear extensions of the first body, which will not be possible if there is a bottle inserted in it due to the limited space between the orifice (15) for inserting the bottle and the first body when the two bodies are not aligned.

With the second body perpendicular to the rear extensions of the first body, the space in which the pill is housed is opened and can be used.

To achieve this swivelling motion, the rear extensions of the first body are provided on their ends with cylindrical protuberances (4) perpendicular to the ones that conform a shaft, while in the internal part of the casing (12) there are some bushings (11) that can act together with the protuberances and facilitate the rotation.

The heating elements are governed by electronic means controlled by the user.

## Claims

1. IMPROVED VAPORISER DEVICE of the type comprising means for vaporising both a substance contained in a bottle and a substance impregnated in a pill, **characterised in that** it comprises:
A first body with means (2) for anchoring it to a surface and means (3) and (4) for allowing its swivelling union to a second body.
A second body with means for allowing its swivelling union to the first body (11), a heatable cavity open to the outside by an orifice (15) with means (7) for attaching a bottle (8) inserted in said cavity, and a heatable cavity open to the outside suitable for inserting and housing a pill.

2. IMPROVED VAPORISER DEVICE according to claim 1, **characterised in that** the means for anchoring the support to a surface comprise electrical connections (2).

3. IMPROVED VAPORISER DEVICE according to claim 1, **characterised in that** the means provided in the first body to allow its swivelling union to a second body comprise extensions in the rear part of the first body (3) and some cylindrical protuberances (4) at its ends disposed perpendicular to the former means.

4. IMPROVED VAPORISER DEVICE according to claim 1, **characterised in that** the means existing in the second body to allow a swivelling union to the first body comprise some bushings (11) that can house the cylindrical protuberances of the ends of the rear extensions of the first body.

5. IMPROVED VAPORISER DEVICE according to claim 1, **characterised in that** the orifice (15) that opens the heating cavity for bottles and the orifice (14) that opens the heating cavity for pills are on opposite faces of the second body.

6. IMPROVED VAPORISER DEVICE according to claim 1, **characterised in that** the cavity for inserting a pill is in the path of the rear extensions of the first body.

7. IMPROVED VAPORISER DEVICE according to claim 1, **characterised in that** when the second body is aligned with the rear extensions of the first body (3), they occupy at least partially the pill heating cavity and the outlet orifice of the bottle heating cavity (15) is in the bottom part of the device.

8. IMPROVED VAPORISER DEVICE according to claim 7, **characterised in that** when the second body is perpendicular to the rear extensions of the first body, the pill heating cavity is free of said extensions and the outlet orifice (15) of the bottle heating cavity is in a lateral position opposite and near to the first body.
